# EUROPÄISCHE PATENTSCHRIFT

(11) **EP 2 857 811 B1**
(45) Veröffentlichungstag und Bekanntmachung des Hinweises auf die Patenterteilung: **23.09.2015**
(21) Anmeldenummer: 13187159.2
(22) Anmeldetag: 02.10.2013
(51) Int. Cl.: G01J 3/02, G01J 3/12, G01J 3/26, G01J 3/433, G01J 3/42, G01N 21/17, G01N 21/35

(54) **Spektrometer zur Gasanalyse**
Spectrometer for gas analysis
Spectromètre pour l'analyse de gaz

(43) Veröffentlichungstag der Anmeldung: 08.04.2015
(73) Patentinhaber: SICK AG, 79183 Waldkirch (DE)
(72) Erfinder: Waldmann, Torsten, 79211 Denzlingen (DE); Disch, Rolf, 79356 Eichstetten (DE)
(74) Vertreter: Ludewigt, Christoph

(56) Entgegenhaltungen:
- EP-A1- 0 543 578
- US-A- 5 357 340

## Beschreibung

Die Erfindung betrifft ein Spektrometer und ein Verfahren zur spektrometrischen Gasanalyse nach dem Oberbegriff von Anspruch 1 beziehungsweise 8.

Zur Messung der Konzentration von Einzelkomponenten eines Gasgemisches sind verschiedene Verfahren der Infrarot-Spektroskopie bekannt. Sie beruhen alle auf dem Grundprinzip, infrarotes Testlicht in eine Messzelle mit dem zu untersuchenden Gas zu strahlen. Jedes Gas zeigt dabei eine charakteristische Absorption bei Frequenzen, die den Spektrallinien seiner Moleküle entsprechen. Diese Absorption verändert die Eigenschaften des eingestrahlten Lichts, das auf verschiedene Arten detektiert und ausgewertet werden kann.

Die Fourier-Transformations-Spektroskopie (FTIR) basiert auf einem Interferometer und der Umrechnung eines damit gewonnenen Interferogramms in ein Spektrum durch Fourier-Transformation. Solche Systeme sind aber kostenintensiv. Gitterspektrographen erzeugen ein Spektrum durch Beugung am Spalt, der jedoch zu einem intensitätsschwachen Signal führt. Bei der nicht-dispersiven Infrarotspektroskopie (NDIR) wird vor dem Detektor ein optisches Bandpassfilter angeordnet, welcher selektiv Licht entsprechend einer zu untersuchenden Spektrallinie durchlässt. Dieses relativ einfache Verfahren ist also unflexibel, da für jede zu erfassende Gaskomponente ein optisches Bandpassfilter vorgesehen sein muss.

Um auch schwache Signale erfassen zu können, werden Lock-In-Verfahren eingesetzt. Das Sendelicht wird also mit einer bekannten Modulationsfrequenz gepulst, und der Empfänger selektiert durch analogen Mischvorgang oder digitale Auswertung das Messsignal anhand der Modulationsfrequenz, um Störer in anderen Frequenzbereichen zu unterdrücken. Die Modulation des Sendelichts wird herkömmlich durch gepulste Lichtquellen oder ihr nachgeordnete mechanische Pulsformer ("Chopper") erzeugt. Beides ist unbefriedigend, weil gepulste Infrarotstrahler nur geringe Modulationsfrequenzen erlauben und ein mechanischer Pulsformer anfällig und unflexibel ist, stabilisiert werden muss und viel Bauraum benötigt.

In EP 2770319 wird vorgeschlagen, das Wellenlängenband des in die Messzelle eingestrahlten Lichts durch ein durchstimmbares Fabry-Pérot-Filter zu verändern und auf diese Weise das Spektrum abzutasten. Ein Fabry-Pérot-Filter ist ein optischer Resonator, der aus zwei teildurchlässigen Spiegeln gebildet wird. Durch konstruktive und destruktive Interferenz werden nur Wellenlängen transmittiert, welche den Resonanzbedingungen entsprechen. Dementsprechend entstehen ein erstes Transmissionsmaximum bei der Resonanzfrequenz und weitere Transmissionsmaxima höherer Ordnung bei den Oberwellen, während die Zwischenfrequenzen nahezu ausgelöscht werden. Der Abstand zwischen den Transmissionsmaxima wird als freier Spektralbereich bezeichnet.

Variiert man den Spiegelabstand beziehungsweise den Luftspalt zwischen den Spiegeln, so werden die Transmissionsmaxima zugleich in ihrer Breite und ihrem gegenseitigen Abstand beeinflusst. Eine feine Nadel eines Transmissionsmaximums bedingt daher einen geringen freien Spektralbereich, während umgekehrt ein großer freier Spektralbereich nur um den Preis von Transmissionsmaxima großer Halbwertsbreite zu erreichen ist. Diese Charakterisierung von Fabry-Pérot-Filtern wird auch in der sogenannten Finesse erfasst, die als Verhältnis des freien Spektralbereichs zu der Halbwertsbreite definiert ist.

Aus diesen Gründen fällt in den Durchlassbereich eines Fabry-Pérot-Filters üblicherweise eine Vielzahl von Absorptionslinien einer oder mehrerer Gaskomponenten, sei es, weil sie innerhalb des gleichen Transmissionsmaximums eines Fabry-Pérot-Filters hoher Halbwertsbreite liegen oder weil der freie Spektralbereich eines Fabry-Perot-Filters geringer Halbwertsbreite zu gering ist, um das Fabry-Pérot-Filter selektiv auf eine Absorptionslinie einzustellen, ohne dass Nebenmaxima mit weiteren Absorptionslinien übereinstimmen.

Das mit einem Fabry-Pérot-Filter erreichbare spektrale Auflösungsvermögen ist daher begrenzt. Möchte man ein höheres spektrales Auflösungsvermögen erreichen, so wird herkömmlich auf die oben genannten Verfahren wie FTIR oder Gitterspektroskopie zurückgegriffen.

Aus der US 5 357 340 ist ein Verfahren zur Spektroskopie unter Verwendung zweier Fabry-Pérot-Filter bekannt. Dabei bleiben die Filtereigenschaften des einen Fabry-Perot-Filters fixiert, so dass es als statisches Etalon wirkt, während der Plattenabstand des anderen Fabry-Pérot-Filters elektrisch oder thermisch variiert wird, um einen Spektralbereich kontinuierlich abzutasten. Zur Modulation der Lichtquelle wird ein mechanischer Strahlzerschneider (Chopper) verwendet.

Die EP 0 543 578 A1 offenbart ein Spektrometer mit einem Filterrad und einem Fabry-Perot-Etalon.

Es ist daher Aufgabe der Erfindung, die Messung eines Spektrums zur Gasanalyse zu verbessern.

Diese Aufgabe wird durch ein Spektrometer und ein Verfahren zur spektrometrischen Gasanalyse nach Anspruch 1 beziehungsweise 8 gelöst. Dabei werden Licht mit Hilfe einer Filteranordnung Frequenzeigenschaften aufgeprägt, ehe es zur Spektrometrie in eine Messzelle mit dem zu untersuchenden Gas beziehungsweise Gasgemisch gestrahlt wird. Die Absorption durch das Gas wird dann detektiert und so die Konzentration von Gaskomponenten gemessen. Die Erfindung geht nun von dem Grundgedanken aus, in der Filteranordnung mehrere Fabry-Pérot-Filter vorzusehen. Durch Verstellen von mindestens einem dieser Fabry-Pérot-Filter verändert sich das Transmissionsspektrum der Filteranordnung, und so können gewünschte Frequenzeigenschaften des Lichts erreicht werden. Das ermöglicht beispielsweise, das Spektrometer nacheinander auf die Absorptionsspektren verschiedener Gase eines Gasgemisches einzustellen oder die Filteranordnung kontinuierlich durchzustimmen, um einen größeren Wellenlängenbereich abzutasten.

Die Erfindung zeigt die Vorteile von Fabry-Pérot-Filtern, die kostengünstig, kompakt, mechanisch einfach und damit wartungsarm und robust sind und die einen hohen optischen Durchsatz haben. Veränderliche Frequenzeigenschaften des eingestrahlten Lichts können sehr schnell und flexibel eingestellt werden. Zugleich ist aber durch die Mehrfachanordnung von Fabry-Pérot-Filtern eine hohe spektrale Auflösung möglich. Es ergeben sich nämlich neue Freiheitsgrade der Verstellung, die beispielsweise genutzt werden können, um zugleich eine geringe Halbwertsbreite und einen großen freien Spektralbereich zu erreichen. Das wäre bei einem einfachen Fabry-Pérot-Filter wegen des direkten Zusammenhangs dieser Größen nicht denkbar und begrenzt die herkömmlich erreichbare spektrale Auflösung erheblich.

Um ein Fabry-Pérot-Filter zu verstellen, wird insbesondere dessen Luftspalt beziehungsweise Spiegelabstand verändert. Dadurch wird die Resonanzfrequenz und infolgedessen das Transmissionsspektrum variiert. Die Fabry-Pérot-Filter sind dafür beispielsweise als elektrostatisch oder piezoelektrisch angesteuerte Mikrosysteme (MEMS, Micro Electro Mechanical System) aufgebaut. Dies vereint Robustheit, kleine Baugrößen und kurze Reaktionszeiten zumindest im Millisekundenbereich.

Die Filteranordnung weist bevorzugt drei Fabry-Pérot-Filter auf. Im Prinzip wird auch schon durch zwei Farby-Pérot-Filter eine höhere Flexibilität gewonnen. Je mehr Fabry-Perot-Filter zusätzlich in der Filteranordnung vorgesehen sind, umso mehr Freiheitsgrade werden gewonnen. Eine Anzahl von drei Farby-Pérot-Filtern bildet dabei einen bevorzugten Kompromiss.

In der Steuereinheit ist bevorzugt eine Zuordnung von Spiegelabständen der Fabry-Perot-Filter und zugehörigem Transmissionsspektrum abgespeichert. Diese Zuordnung wird ab Werk vorgegeben oder anfänglich in Kalibrationsmessungen eingelernt. Anhand der Zuordnung kann die Steuereinheit ein gewünschtes Transmissionsspektrum der Filteranordnung einstellen. An einem Verstellvorgang ist mindestens ein Fabry-Perot-Filter beteiligt, es können aber auch mehrere oder alle sein.

Die Fabry-Pérot-Filter weisen bevorzugt gestaffelte Halbwertsbreiten auf, insbesondere ist bei drei Fabry-Pérot-Filtern jeweils ein Fabry-Pérot-Filter mit geringer, mittlerer und hoher Halbwertsbreite vorgesehen ist. Intuitiv am verständlichsten ist ein Zusammenspiel der Fabry-Pérot-Filter, bei welchem das Fabry-Pérot-Filter geringster Halbwertsbreite die spektrale Auflösung bestimmt und die übrigen Fabry-Pérot-Filter gestaffelt dafür sorgen, dass die höheren Ordnungen des jeweils feiner auflösenden Fabry-Perot-Filters ausgeblendet werden. Es gibt aber in vielen Situationen auch andere Kombinationen, um ein gewünschtes Transmissionsspektrum zu erzielen.

Das Transmissionsspektrum weist bevorzugt ein schmalbandiges Maximum bei einer vorgebbaren Frequenz auf. Zusätzlich sind noch bevorzugter Frequenzanteile außerhalb dieses Maximums weitestgehend unterdrückt. So sorgt die Filteranordnung dafür, dass nur Licht mit einer bestimmten Frequenz in die Messzelle gelangt. Mit mehreren Einzelmessungen unter zwischenzeitlichem Verschieben der Frequenz des Maximums kann so ein Spektrum abgetastet werden.

Die Steuereinheit ist bevorzugt dafür ausgebildet, das Transmissionsspektrum der Filteranordnung derart zyklisch zu variieren, dass die Amplitude des Maximums moduliert wird. Auf diese Weise bildet die Filteranordnung zugleich einen optischen Pulsformer. Das gelingt insbesondere durch ein aufeinander abgestimmtes zyklisches Verstellen von Fabry-Pérot-Filtern. Beispielsweise wird das Maximum des Fabry-Pérot-Filters mit der größten Halbwertsbreite zyklisch so verschoben, dass die Lage dieses Maximums in unterschiedlichem Maße mit den Maxima der anderen Fabry-Pérot-Filter übereinstimmt. Dadurch wird eine ähnliche Wirkung erzielt wie durch eine Blende, die das Licht alternativ durchlässt oder blockt. Es gibt aber auch zahlreiche andere Stellungen der Fabry-Pérot-Filter, in denen sich deren Transmissionsmaxima kaum oder nicht überlagern, so dass das Licht praktisch geblockt wird, und die auch in Zwischenstufen angefahren werden können, um auch andere als reine Hell-Dunkel-Modulationen zu erreichen. Die Vielzahl von Fabry-Pérot-Filtern ermöglicht also in einer Doppelfunktion, das Spektrum mit hoher Auflösung abzutasten und gleichzeitig ohne zusätzliches Element eine Modulation der in die Messzelle fallenden Lichtintensität zu erreichen.

Die Amplitude wird bevorzugt mit einer vorgebbaren Funktion moduliert, insbesondere einem Deltapuls, einem Sinus oder einem exponentiell anwachsenden und dann abrupt abfallenden Puls. Ein Deltapuls entspricht einer reinen Hell-Dunkel-Modulation. Ein Sinus beziehungsweise Cosinus unterstützt elektronische und digitale Lock-In-Verfahren besonders gut. Die Funktion lässt sich aber nahezu beliebig wählen, indem die Filteranordnung in Zwischenstellungen verfahren wird, welche zu unterschiedlichen Dämpfungen des Transmissionsmaximums führen. Eines von zahlreichen Beispielen ist eine Modulation mit Pulsen, die ähnlich aussehen wie Haifischflossen, d.h. zunächst vergleichsweise langsam exponentiell auf ihr Helligkeitsmaximum anwachsen und dann plötzlich in den Dunkelzustand zurückfallen. Eine solche Modulation bildet ein einfach zu rechnendes Beispiel.

Die Steuereinheit ist bevorzugt dafür ausgebildet, das Transmissionsspektrum nach einem Abtastschema zu verändern, nach dem jeweils ein Transmissionsspektrum mit einem Maximum bei einer Abtastfrequenz erzeugt, die Amplitude des Maximums bei der Abtastfrequenz über mehrere Zyklen moduliert und die Abtastfrequenz dann systematisch verändert wird. Mit einem solchen Abtastschema wird das Spektrum nacheinander bei verschiedenen Frequenzen jeweils mit einer Pulsfolge abgetastet.

Ein Signal des Detektors wird vorzugsweise mit einem Lock-In-Verfahren anhand der bekannten Modulation ausgewertet. Dies ist das Prinzip des Lock-In-Verstärkers, welcher sich analog oder digital auf die bekannte Modulationsfrequenz einstellt und dadurch ein wesentlich besseres Signal-Rausch-Verhältnis erzielt. Die erforderliche Modulation des Lichts erfolgt vorzugsweise wie soeben beschrieben in der Filteranordnung selbst, aber prinzipiell ist auch ein zusätzlicher optischer Pulsformer oder eine gepulste Lichtquelle denkbar.

In dem Lichtweg ist bevorzugt ein zusätzliches optisches Bandpassfilter vorgesehen, um Maxima höherer Ordnungen zumindest eines der Fabry-Pérot-Filter auszublenden, vorzugsweise des Fabry-Pérot-Filters des höchsten freien Spektralbereichs. Schon innerhalb der Filteranordnung erfüllen die Fabry-Pérot-Filter mit größerem freiem Spektralbereich praktisch diese Funktion, indem sie höhere Ordnungen von Fabry-Perot-Filtern eines kleineren freien Spektralbereichs unterdrücken. Das reicht aber nicht unbedingt aus, um alle unerwünschten Spektralanteile auszuschließen, und deshalb begrenzt das optische Bandpassfilter das Frequenzband insgesamt auf einen interessierenden Bereich, der vorzugsweise höchstens so groß ist wie der resultierende freie Spektralbereich der Filteranordnung. Ein solches Bandpassfilter kann auch in eine Sende- oder Empfangsoptik in dem Lichtweg integriert werden. Alternativ sind Lichtquelle oder Detektor schmalbandig genug, um verbleibende höhere Ordnungen der Filteranordnung von sich aus auszuschließen.

Die Messzelle ist bevorzugt eine photoakustische Messzelle. Darin können auch geringe Gaskonzentrationen bei kurzen Absorptionsstrecken gemessen werden, und die Messzelle hat einen hohen dynamischen Bereich. Die photoakustische Messzelle misst Druckänderungen, die durch Erwärmung des Gases bei Strahlungsabsorption des in die Messzelle eingestrahlten Lichts erzeugt werden. Diese Druckänderungen werden akustisch beispielsweise mit einem Mikrophon oder Drucksensor gemessen. Vorzugsweise sorgt hierbei moduliertes Licht für genügend Zustandsänderungen.

In einer bevorzugten Ausführungsform der photoakustischen Messzelle weist dies einen Schwingbalken (Cantilever) etwa in Form einer Siliziummembran und ein Interferometer zur optischen Messung der Auslenkung des Schwingbalkens mittels Interferometrie auf. Die Absorption sorgt bei ausreichender Übereinstimmung der Frequenzeigenschaften des eingestrahlten Lichts und des Absorptionsspektrums einer Gaskomponente für eine sehr schnelle Erwärmung und damit eine impulsartige Druckänderung des Gases. Die dadurch bewirkte Auslenkung des Schwingbalkens wird dann interferometrisch ausgewertet.

Vorzugsweise ist eine Kalibriereinheit vorgesehen, die in den Lichtweg einführbar und daraus entfernbar ist und die einen Interferenzfilter mit einer bekannten Referenzfrequenz oder eine Gasküvette mit einem Referenzgas aufweist. Obwohl die Resonanzeigenschaften eines Fabry-Pérot-Filters und damit dessen Transmissionsspektrum theoretisch sehr gut vorhergesagt werden kann, ergeben sich in der Praxis Toleranzen, wenn die Steuereinheit einen bestimmten Spiegelabstand einstellt. Dies wird in einer Kalibration durch Vergleich mit der bekannten Referenzfrequenz ausgeglichen und so die jeweilige Durchlasswellenlänge und/oder Intensität besonders genau einstellbar. Das Referenzgas kann auch in die Messzelle eingeführt werden. Bei Verwendung eines Referenzgases ist wie üblich auf temperatur- und druckstabile Verhältnisse zu achten.

Die Kalibriereinheit weist bevorzugt einen Verfahrschlitten oder ein Filterrad auf. Dadurch kann die Kalibration einfach durchgeführt und bei Bedarf wiederholt werden. Es können jeweils mehrere Referenzgase beziehungsweise Interferenzfilter vorgesehen sein, um mehrere Kalibrationspunkte zu eichen.

Mindestens ein Fabry-Pérot-Filter ist bevorzugt aus dem Lichtweg entfernbar und wieder in den Lichtweg einbringbar montiert. Auch dazu kann ein Verfahrschlitten dienen. Die Fabry-Pérot-Filter können auf diese Weise nicht nur gemeinsam als Filteranordnung, sondern individuell oder in Gruppen kalibriert werden. Das vereinfacht den Kalibrationsvorgang und führt zu genaueren Ergebnissen.

Das erfindungsgemäße Verfahren kann auf ähnliche Weise weitergebildet werden und zeigt dabei ähnliche Vorteile. Derartige vorteilhafte Merkmale sind beispielhaft, aber nicht abschließend in den sich an die unabhängigen Ansprüche anschließenden Unteransprüchen beschrieben.

Die Erfindung wird nachstehend auch hinsichtlich weiterer Merkmale und Vorteile beispielhaft anhand von Ausführungsformen und unter Bezug auf die beigefügte Zeichnung näher erläutert. Die Abbildungen der Zeichnung zeigen in:
- Fig. 1: eine Übersichtsdarstellung eines Spektrometers;
- Fig. 2a: Transmissionsspektren von drei unterschiedlichen Fabry-Pérot-Filtern;
- Fig. 2b: ein gemeinsames Transmissionsspektrum der drei hintereinander angeordneten Fabry-Pérot-Filter gemäß Figur 2a;
- Fig. 3: eine beispielhafte spektrale Abtastung von CO;
- Fig. 4a: Transmissionsspektren ähnlich Figur 2a von drei unterschiedlichen Fabry-Perot-Filtern, jedoch mit gegenseitigem Verstimmen, damit die Transmissionsmaxima einen gegenseitigen Versatz erhalten;
- Fig. 4b: ein gemeinsames Transmissionsspektrum ähnlich Figur 2b der drei hintereinander angeordneten Fabry-Pérot-Filter gemäß Figur 4a;
- Fig. 5a: Transmissionsspektren gemäß Figur 2a und 4a zur Illustration der möglichen Einstelländerungen zur Amplitudenmodulation des resultierenden Maximums in dem gemeinsamen Transmissionsspektrum;
- Fig. 5b: das Maximum des gemeinsamen Transmissionsspektrums der drei hintereinander angeordneten Fabry-Pérot-Filter gemäß Figur 5a bei mehreren Einstelländerungen;
- Fig. 6: ein Abtastschema zur Modulation des eingestrahlten Lichts mit jeweils fünf Hell-Dunkel-Zyklen je eingestellter Durchlasswellenlänge;
- Fig. 7: ein weiteres Abtastschema zur Modulation des eingestrahlten Lichts mit jeweils zwei haifischflossenartigen Zyklen je eingestellter Durchlasswellenlänge;
- Fig. 8: eine Übersichtsdarstellung einer weiteren Ausführungsform des Spektrometers mit variabel in den Lichtweg einbringbaren Elementen zur Kalibration; und
- Fig. 9: eine Übersichtsdarstellung einer weiteren Ausführungsform eines Spektrometers mit wahlweise aus dem Lichtweg entfernbaren Fabry-Pérot-Filtern.

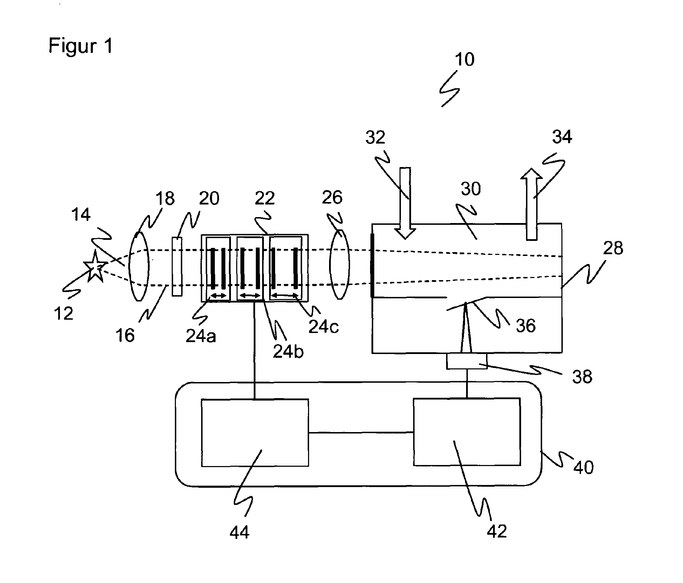

Figur 1 zeigt eine Übersichtsdarstellung eines Spektrometers 10 zur Bestimmung der Konzentration von Gaskomponenten mittels Absorptionsmessung. Eine Lichtquelle 12 sendet Licht 14 aus, das auf seinem Lichtweg 16 eine erste Optik 18, ein Bandpassfilter 20, eine Filteranordnung 22 mit mehreren Fabry-Pérot-Filtern 24a-c und eine zweite Optik 26 passiert und dann in eine Messzelle 28 eingestrahlt wird. In der Messzelle 28 befindet sich das zu untersuchende Gas 30 beziehungsweise ein Gasgemisch, für das wie durch Pfeile symbolisiert ein Gaseinlass 32 und ein Gasauslass 34 vorgesehen sind.

In der Messzelle 28 befindet sich ein Detektor, der hier als Schwingbalken 36 mit einem (Laser-)Interferometer 38 zur Bestimmung der Auslenkung des Schwingbalkens 36 ausgebildet ist. Eine Steuer- und Auswertungseinheit 40 weist eine Messeinheit 42 zum Auswerten der Signale des Interferometers 38, eine Steuereinheit 44 zum Verstellen der Filteranordnung 22 und die übrige notwendige Steuer- und Auswertungsfunktionalität auf. Die Aufteilung der Steuer- und Auswertungseinheit 40 dient nur der anschaulichen Erläuterung, es ist auch jede andere Verteilung auf eine oder mehrere physische Steuer- und Auswertungseinheiten möglich, die untereinander Daten und Zustandsparameter austauschen.

Die Lichtquelle 12 ist vorzugsweise ein breitbandiger Infrarotstrahler. Damit wird der typische Frequenzbereich von Spektrallinien der Moleküle zu untersuchender Gase abgedeckt. Das Licht 12 wird von der ersten Optik 18 gebündelt, wobei die dargestellte Linse nur stellvertretend für eines oder mehrere geeignete optische Elemente zur Strahlformung und Strahlführung steht. Das Bandpassfilter 20 schneidet aus dem breiten Frequenzband der Lichtquelle 12 einen groben Bereich heraus, innerhalb dessen Absorptionsbänder der zu untersuchenden Gase liegen. Beispielsweise können in dem Frequenzband von 4,3 µm bis 7,4 µm die Gaskomponenten CO, NO, SO₂, NO₂, N₂O, CO₂ und H₂O gemessen werden und in dem Frequenzband von 3 µm bis 3,8 µm zahlreiche Kohlenwasserstoffe. Frequenzen außerhalb solcher Bereiche, oder Frequenzen mit Spektrallinien von Gasen, die mit einem bestimmten Spektrometer gar nicht gemessen werden sollen, können deshalb von vorneherein mit Hilfe des Bandpassfilters 20 unterdrückt werden. Das Bandpassfilter 20 kann sich auch an einer anderen Position in dem Lichtweg 16 befinden als dargestellt oder in eine der Optiken 18, 26 integriert werden. Alternativ wird eine etwas schmalbandigere Lichtquelle 12 eingesetzt, etwa eine LED, und auf das Bandpassfilter 20 verzichtet. Auch die Filteranordnung 22 selbst kann zumindest in Grenzen die Funktion des Bandpassfilters 20 übernehmen.

Das Transmissionsspektrum der Filteranordnung 22 kann durch Verstellen der Fabry-Perot-Filter 24a-c, die weiter unten detailliert betrachtet wird, in einer gewünschten Weise vorgegeben werden, so dass das Licht 14 nach Passieren der Filteranordnung 22 bestimmte Frequenzeigenschaften hat. Vorzugsweise verbleibt nur ein schmales Transmissionsmaximum bei einer vorgegebenen Frequenz, das dann in mehreren Einzelmessungen durch den interessierenden Frequenzbereich verschoben wird. Alternativ wird das Transmissionsmaximum gezielt mit einer bekannten Spektrallinie in Übereinstimmung gebracht, um die zugehörige Gaskomponente zu vermessen. Die zweite Optik 26 leitet das gefilterte Licht 14 in die Messzelle 28, wobei wie schon bei der ersten Optik 18 die Darstellung als einfache Linse rein beispielhaft zu verstehen ist. Auch innerhalb der Filteranordnung 22 können weitere optische Elemente insbesondere zwischen den Fabry-Pérot-Filtern 24a-c vorgesehen sein.

Nur wenn die Frequenzeigenschaften des in die Messzelle 28 eingestrahlten Lichts mit einer oder mehreren Spektrallinien der Moleküle des Gases 30 übereinstimmen, wird Licht entsprechender Frequenz absorbiert. Dies führt dann zu einer Erwärmung des Gases 30 und damit einer Druckerhöhung in der Messzelle 28, was wiederum den Schwingbalken 36 proportional zur Konzentration der betreffenden Gaskomponente oder Gaskomponenten auslenkt. Das Interferometer 38 erkennt diese Auslenkung mit hoher Präzision. Die Messung mit einer photoakustischen Messzelle 28 dieser Bauart ist deshalb besonders genau. Es kann aber auch eine andere photoakustische Messung, etwa durch Mikrophon oder Drucksensor, sowie eine gänzlich andere Detektion beispielsweise über einen Infrarotdetektor vorgesehen sein, welcher das durch das Gas 30 transmittierte Licht 14 misst und daraus auf die Absorption geschlossen wird. Dabei sind Anordnungen mit einem Detektor außerhalb der Messzelle 28 denkbar, und das durch die Messzelle 28 transmittierte Licht wird dann mittels einer geeigneten Optik auf den Detektor gelenkt. Ebenfalls möglich ist eine Anordnung, bei der das Licht hinter der Messzelle 28 reflektiert und dann nach doppelter Transmission durch die Messzelle 28 in einem Detektor registriert wird, etwa mittels Strahlteileranordnung zwischen Lichtquelle 12 und Detektor.

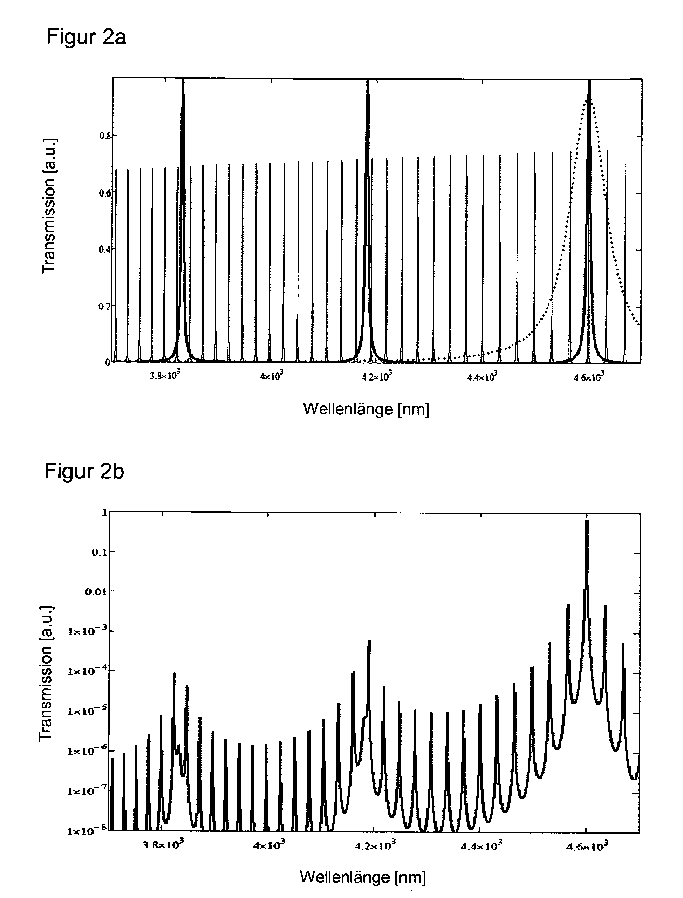

Unter Bezugnahme auf die Figuren 2 bis 7 wird nun die Funktionsweise der Filteranordnung 22 näher erläutert. Die Steuereinheit 44 ist in der Lage, Parameter zumindest eines der Fabry-Pérot-Filter 24a-c zu verändern, insbesondere den Luftspalt zwischen dessen beiden halbdurchlässigen Spiegeln und damit die Resonanzfrequenz. Das kann etwa durch elektrostatische oder piezoelektrische Ansteuerung von als MEMS ausgebildeten Fabry-Pérot Filtern mit kurzen Reaktionszeiten ohne makroskopische Bewegungen umgesetzt werden. Dadurch kann das Transmissionsspektrum der Filteranordnung 22 vorgegeben werden.

Figur 2a zeigt ein Beispiel dreier Transmissionsspektren von drei Fabry-Pérot-Filtern 24a-c. Ein Fabry-Pérot-Filter 24a kleinster Halbwertsbreite erzeugt die mit dünner Linie gezeigten eng beieinanderliegenden nadelartigen Transmissionsmaxima. Ein weiteres Fabry-Pérot-Filter 22b mittlerer Halbwertsbreite weist in dem dargestellten Frequenzausschnitt drei mit fetter Linie gezeigte Transmissionsmaxima. Ein drittes Fabry-Perot-Filter 24c hat in dem dargestellten Frequenzausschnitt nur ein relativ breites mit gepunkteter Linie gezeigtes Transmissionsmaximum. Dies illustriert auch die einleitend beschriebene Abhängigkeit von Halbwertsbreite und Dichte der Transmissionsmaxima, d.h. freiem Spektralbereich: je schmaler und damit für höhere Spektralauflösung geeigneter ein Transmissionsmaximum, desto näher liegt auch das nächste Transmissionsmaximum höherer Ordnung. Keines der Fabry-Pérot-Filter 24a-c einzeln eignet sich daher für eine hochauflösende Spektrometrie, weil das Licht nicht gezielt auf einen bestimmten schmalen Frequenzbereich beschränkt wird.

Das wird durch Hintereinanderordnung der drei Fabry-Pérot-Filter 24a-c gelöst. Figur 2b zeigt das zugehörige gemeinsame Transmissionsspektrum, also letztlich die punktweise Multiplikation der drei Transmissionsspektren aus Figur 2a. Es ergibt sich ein deutlich ausgeprägtes Transmissionsmaximum an der Stelle, wo sich in Figur 2a Transmissionsmaxima aller drei Fabry-Pérot-Filter 24a-c überlagern. Die übrigen Transmissionsmaxima sind noch erkennbar, aber sehr deutlich unterdrückt, weil jeweils ein Fabry-Pérot-Filter 24a-c mit größerem freiem Spektralbereich die Transmissionsmaxima höherer Ordnung eines Fabry-Pérot-Filters mit schmaler Halbwertsbreite unterdrückt. Dabei ist auch die logarithmische Darstellung der Figur 2b zu beachten. Auch bei dem Fabry-Pérot-Filter 24c mit dem breitesten Transmissionsmaximum genügt der freie Spektralbereich nicht zwingend, um die gesamte Bandbreite der Lichtquelle 12 abzudecken, wofür optional das Bandpassfilter 20 den Frequenzbereich zusätzlich verkleinert, beispielsweise auf den in den Figuren gezeigten Ausschnitt oder allgemein auf einen Durchlassbereich, der höchstens so groß ist wie der freie Spektralbereich eines oder mehrerer der Fabry-Pérot-Filter 24a-c.

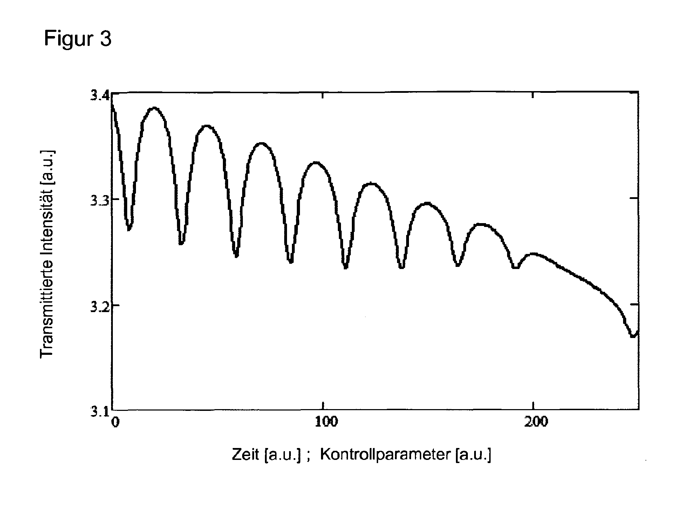

Durch sukzessives Verändern der zentralen Wellenlänge des in Figur 2b gezeigten Bereichs kann nun das Absorptionsspektrum des Gases 30 abgetastet werden. Figur 3 zeigt beispielhaft das Ergebnis der sukzessiven Abtastung des Spektrums von CO.

Diese hochauflösende Spektrometrie kann nun mit gleichem Aufbau noch um eine Modulation erweitert werden, um in der Detektion ein Lock-In-Verfahren zu ermöglichen. In der konkret gezeigten Ausführungsform mit der photoakustischen Messzelle 28 wird dadurch der Schwingbalken 24 periodisch ausgelenkt und dessen Schwingung interferometrisch ausgewertet. Lock-In-Verfahren können aber auch sonst, beispielsweise zur Verstärkung des Signals eines Infrarotdetektors verwendet werden.

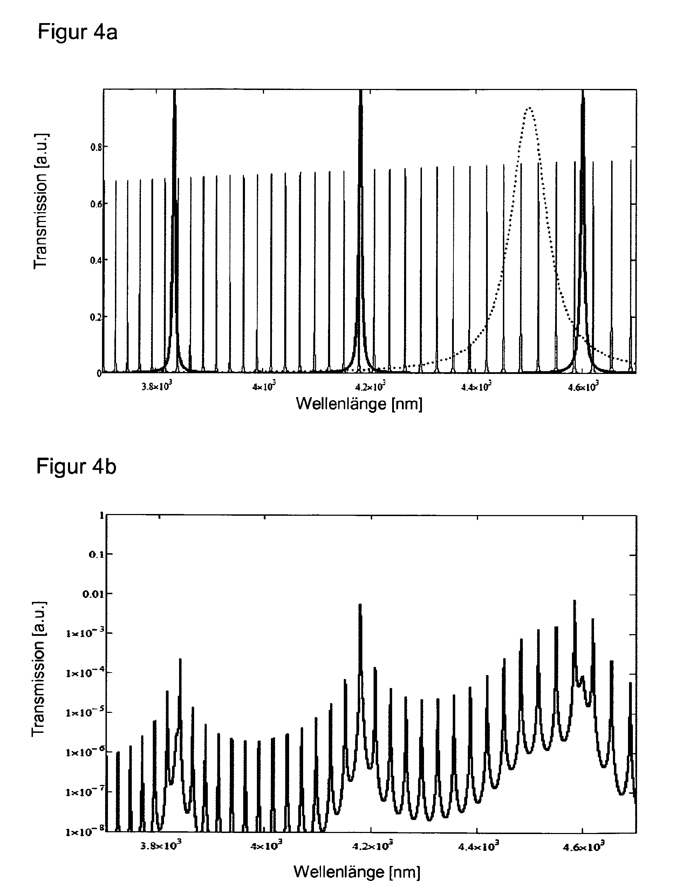

Die Filteranordnung 22 ist in einer Doppelfunktion selbst und damit ohne zusätzliches Bauteil in der Lage, die erforderliche Modulation zu erzeugen. Dazu werden die Fabry-Perot-Filter 24a-c periodisch gegeneinander verstimmt und damit gezielt der in Figur 2b gezeigte Hell-Zustand vorübergehend verlassen. Dies ist in Figur 4 illustriert. Figur 4a zeigt eine ähnliche Darstellung wie Figur 2a, wobei hier aber die Transmissionsmaxima gegeneinander verschoben sind, so dass sie sich möglichst bei keiner Frequenz überlagern. Für das breiteste, in gepunkteter Linie gezeigte Transmissionsmaximum ist das sofort erkennbar. Aber auch das in fetter Linie gezeigte mittlere Transmissionsmaximum stimmt bei 4,6 µm nicht mehr mit einem in dünner Linie gezeigten nadelartigen Transmissionsmaximum überein. Gegebenenfalls ließe sich auch durch Feineinstellung noch die Überlagerung bei 4,2 µm verhindern. In dem resultierenden gemeinsamen Transmissionsspektrum gemäß Figur 4b ist das Transmissionsmaximum bei 4,6 µm um zwei Größenordnungen gedämpft. Ein zyklischer Wechsel zwischen den Einstellungen gemäß Figur 2 und Figur 4 führt daher zu einer Hell-Dunkel-Modulation mit zwei Helligkeitsstufen pro Durchlasswellenlänge.

Es sind aber nicht nur einfache Hell-Dunkel-Modulationen, sondern wählbare, zeitlich variable Modulationen der transmittierten Lichtleistung möglich. Dazu wird nicht unmittelbar ein Dunkel-Zustand angefahren wie in Figur 4, sondern auch Zwischenzustände verschiedener Intensität.

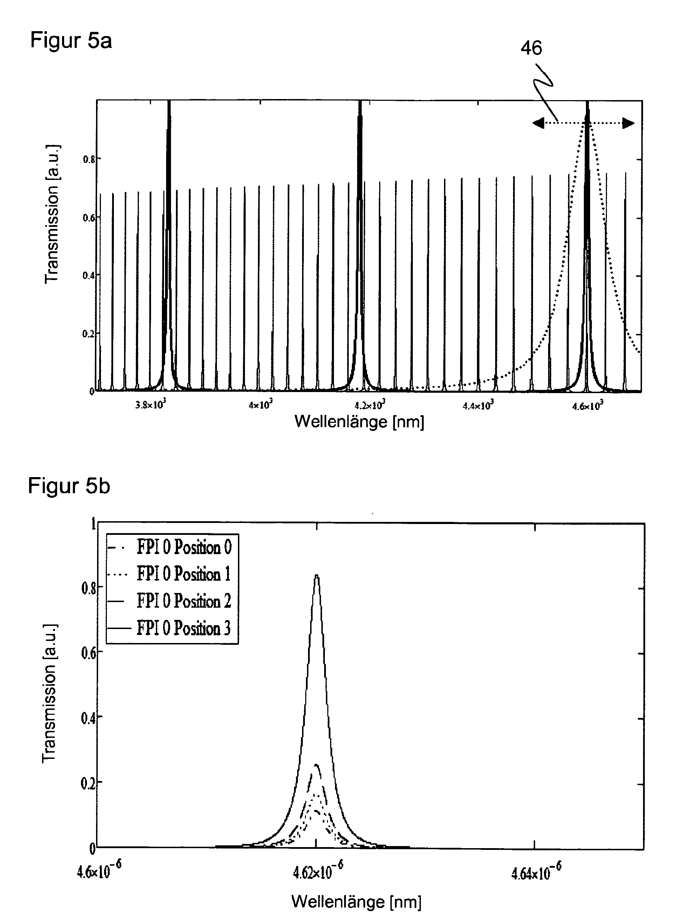

Figur 5 erläutert das Vorgehen. Die Darstellung der Figur 5a entspricht im Wesentlichen der Figur 2a, es wird aber durch einen Pfeil 46 ein Modulationsschema angedeutet. Dieses liegt darin, den Luftspalt des Fabry-Pérot-Filters 24c mit größter Halbwertsbreite und größtem freiem Spektralbereich zu verstellen. Das ist nur ein besonders intuitiv verständliches Beispiel, auch andere Kombinationen von Einstellungen der Fabry-Pérot-Filter 24a-c würden zu dem gewünschten Ergebnis führen.

In den Zwischenzuständen ergibt sich für das gemeinsame Transmissionsmaximum bei 4,6 µm eine unterschiedliche Amplitude. Das ist in Figur 5b beispielhaft für vier Stellungen des Fabry-Pérot-Filters 24c gezeigt.

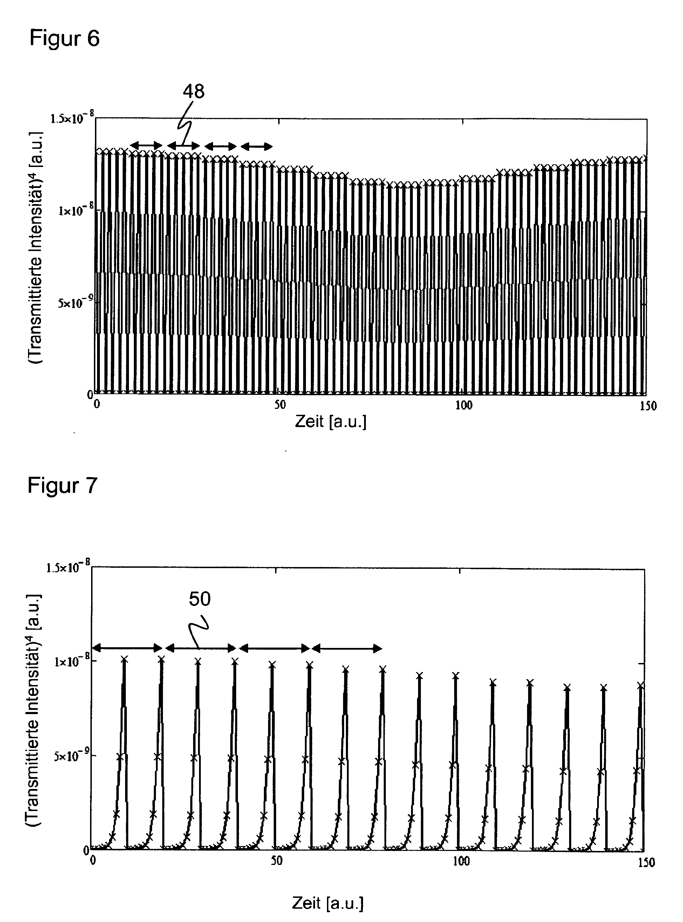

Figur 6 zeigt einen Ausschnitt einer beispielhaften Abtastung einer CO-Absorptionslinie mit einem Abtast- und Modulationsschema. Bei jeder eingestellten Durchlasswellenlänge werden jeweils fünf Hell-Dunkel-Zyklen durchgeführt. Jeweils zehn Datenpunkte entsprechen also einer Durchlasswellenlänge. Die Zyklen bei vier Durchlasswellenlängen sind durch Pfeile 48 illustriert.

Sowohl Anzahl der Zyklen als auch die Modulation je Zyklus sind beliebig variierbar. Figur 7 zeigt dazu einen Auszug einer weiteren beispielhaften Abtastung einer CO-Linie mit einem anderen Abtast- und Modulationsschema. Hier werden bei jeder eingestellten Durchlasswellenlänge jeweils zwei Modulationszyklen durchgeführt, wie für vier Zyklen durch Pfeile 50 illustriert. Die Modulation selbst ist kein einfacher Hell-Dunkel-Wechsel mehr, sondern führt über mehrere Schritte, hier insgesamt zehn Intensitätsmodulationen, mit einem exponentiellen Anwachsen und anschließenden abrupten Abfallen ("Haifischflosse"). In analoger Weise lassen sich praktisch beliebige Abtast- und Modulationsschemata verwirklichen.

Die Filteranordnung 22 ist somit in der Lage, Licht in einem schmalen Wellenlängenbereich um eine sich sukzessive ändernde Zentralwellenlänge in die Messzelle 28 zu transmittieren, wobei das Licht bei Bedarf auch moduliert werden kann.

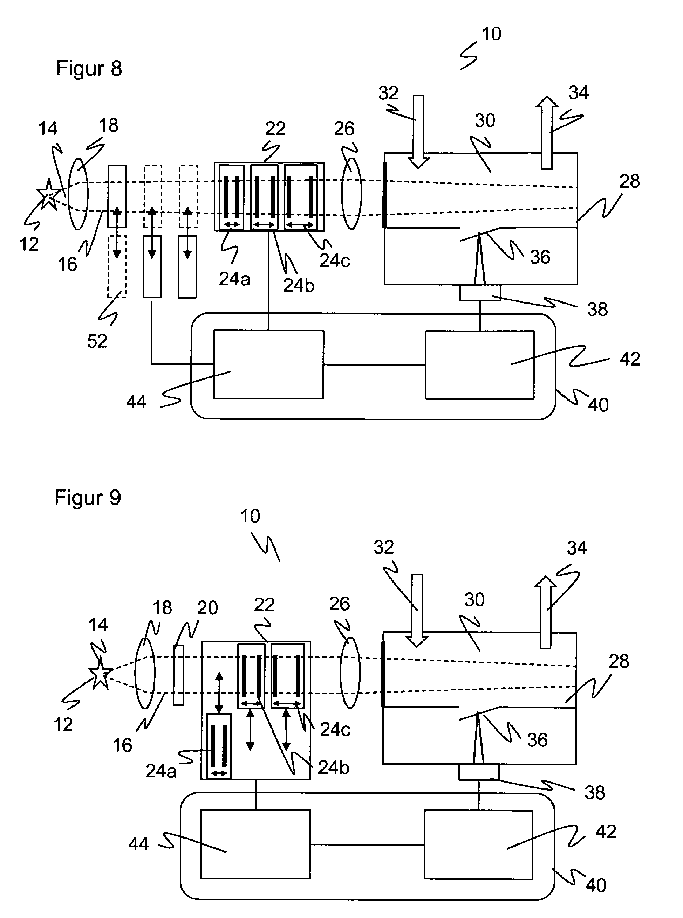

Das Transmissionsspektrum eines Fabry-Pérot-Filters 24a-c hängt von Kontrollparametern ab, die von der Steuereinheit 44 eingestellt werden können. Um dies zu kalibrieren, können in einer weiteren Ausführungsform des Spektrometers 10 gemäß Figur 8 zusätzliche Elemente 52 als Kalibriereinheit in den Lichtweg 16 eingebracht beziehungsweise daraus entfernt werden. Dabei haben die Elemente 52 ein bekanntes Spektrum, das als Referenz herangezogen wird. Zwei Beispiele solcher Elemente 52 sind Interferenzfilter bekannter wellenlängenabhängiger Transmission oder abgeschlossene Referenzgasküvetten bekannter Länge und Füllung mit einer oder mehreren Gaskomponenten bekannter Konzentration. Dabei sollte auf Temperatur- und im Falle eines Referenzgases auch Druckstabilität geachtet werden. Unter Verwendung der Elemente 52 und Vermessung des Spektrums werden die Transmissionsspektren einschließlich Durchlasswellenlänge und Transmissionsintensität der Fabry-Pérot-Filter 24a-c abhängig von einem oder mehreren Kontrollparametern kalibriert. Das Einbringen oder Herausnehmen der Elemente 52 kann durch ein Filterrad oder durch mechanische Verfahrschlitten erreicht werden. Eine alternative Ausführungsform sieht anstelle von Elementen 52 in dem Lichtweg 16 eine schmalbandige Laserlichtquelle zur Kalibration vor.

Figur 9 zeigt eine weitere Ausführungsform des Spektrometers 10. In dieser Ausführungsform sind die Fabry-Perot-Filter 24a-c ebenfalls über Verfahrschlitten oder dergleichen einzeln oder in Gruppen aus dem Lichtweg 16 entfernbar und in diesen einbringbar. Dies lässt sich auch mit der Ausführungsform gemäß Figur 8 kombinieren. Die Ausführungsform gemäß Figur 9 erlaubt, die Fabry-Pérot-Filter 24a-c einzeln oder in Gruppen und nicht nur die Filteranordnung 22 in ihrer Gesamtheit zu kalibrieren. Das erhöht die Genauigkeit und erleichtert das Vorgehen.

Zur Kalibration der Fabry-Pérot-Filter 24a-c ist auch denkbar, die Messzelle 28 mit einem Kalibrations- oder Referenzgas zu füllen, dessen Gaskomponenten einschließlich deren Konzentration bekannt sind. Weiterhin ist möglich, zur Kalibration anstelle der Messzelle 28 einen Detektor in den Lichtweg 16 einzubringen, welcher die Intensität im relevanten Spektralbereich messen und dadurch die Einstellung der Filteranordnung 22 beziehungsweise die Eigenschaften des gesamten Lichtwegs 16 überprüfen kann.

## Patentansprüche

1. Spektrometer (10) zur Gasanalyse, das eine Messzelle (28) mit einem zu untersuchenden Gas (30), eine Lichtquelle (12) zum Aussenden von Licht (14) in die Messzelle (28) auf eine Lichtweg (16), eine Filteranordnung (22) mit einem Fabry-Pérot-Filter (24a-c) in dem Lichtweg (16), um durch das Transmissionsspektrum der Filteranordnung (22) Frequenzeigenschaften des Lichts (14) einzustellen, sowie einen Detektor (36, 38) aufweist, welcher die Absorption des Lichts (14) durch das Gas (30) in der Messzelle (28) misst, wobei die Filteranordnung (22) mehrere hintereinander in dem Lichtweg (14) angeordnete Fabry-Perot-Filter (24a-c) und eine Steuereinheit (44) für die Filteranordnung (22) aufweist, um das Transmissionsspektrum durch Verstellen der Fabry-Perot-Filter (24a-c) zu verändern,
**dadurch gekennzeichnet,**
**dass** die Steuereinheit (44) dafür ausgebildet ist, das Transmissionsspektrum durch Verstellen der Fabry-Pérot-Filter (24a-c) nach einem Abtastschema zu verändern, nach dem jeweils ein Transmissionsspektrum mit einem schmalbandigen Maximum bei einer Abtastfrequenz erzeugt, die Amplitude des Maximums bei der Abtastfrequenz über mehrere Zyklen moduliert und die Abtastfrequenz dann systematisch verändert wird, so dass das Spektrum nacheinander bei verschiedenen Frequenzen jeweils mit einer Pulsfolge abgetastet wird, wobei das Signal des Detektors mit einem Lock-In-Verfahren anhand der bekannten Modulation ausgewertet wird.

2. Spektrometer (10) nach Anspruch 1,
wobei die Filteranordnung (22) drei Fabry-Pérot-Filter (24a-c) aufweist.

3. Spektrometer (10) nach Anspruch 1 oder 2,
wobei in der Steuereinheit (44) eine Zuordnung von Spiegelabständen der Fabry-Pérot-Filter (24a-c) und zugehörigem Transmissionsspektrum abgespeichert ist.

4. Spektrometer (10) nach Anspruch 2 oder 3,
wobei die Fabry-Pérot-Filter (24a-c) gestaffelte Halbwertsbreiten aufweisen, so dass jeweils ein Fabry-Pérot-Filter (24a-c) mit geringer, mittlerer und hoher Halbwertsbreite vorhanden ist.

5. Spektrometer (10) nach einem der vorhergehenden Ansprüche,
wobei die Amplitude mit einem Deltapuls, einem Sinus oder einem exponentiell anwachsenden und dann abrupt abfallenden Puls moduliert wird.

6. Spektrometer (10) nach einem der vorhergehenden Ansprüche,
wobei in dem Lichtweg (16) ein zusätzliches optisches Bandpassfilter (20) vorgesehen ist, um Maxima höherer Ordnungen zumindest eines der Fabry-Perot-Filter (24a-c) auszublenden.

7. Spektrometer (10) nach einem der vorhergehenden Ansprüche,
wobei mindestens ein Fabry-Pérot-Filter (24a-c) aus dem Lichtweg (16) entfernbar und wieder in den Lichtweg (16) einbringbar montiert ist.

8. Verfahren zur spektrometrischen Gasanalyse, bei dem Licht (14) einer Lichtquelle (12) durch eine Filteranordnung (22) mit einem Fabry-Pérot-Filter (24a-c) tritt und dabei die Frequenzeigenschaften des Lichts (14) durch das Transmissionsspektrum der Filteranordnung (22) eingestellt werden, das Licht (14) anschließend in eine Messzelle (28) mit einem zu untersuchenden Gas (30) geführt wird und die Absorption des Lichts (14) durch das Gas (30) in der Messzelle (28) mittels eines Detektors (36, 38) gemessen wird, wobei die Absorption nach jeweiligem Verstellen eines von mehreren hintereinander angeordneten Fabry-Pérot-Filtern (24a-c) der Filteranordnung (22) und dadurch mit Licht unterschiedlicher Frequenzeigenschaften mehrfach gemessen wird,
**dadurch gekennzeichnet,**
**dass** das Transmissionsspektrum durch Verstellen der Fabry-Perot-Filter (24a-c) mittels einer Steuereinheit (44) nach einem Abtastschema verändert wird, nach dem jeweils ein Transmissionsspektrum mit einem schmalbandigen Maximum bei einer Abtastfrequenz erzeugt, die Amplitude des Maximums bei der Abtastfrequenz über mehrere Zyklen moduliert und die Abtastfrequenz dann systematisch verändert wird, so dass das Spektrum nacheinander bei verschiedenen Frequenzen jeweils mit einer Pulsfolge abgetastet wird, wobei das Signal des Detektors mit einem Lock-In-Verfahren anhand der bekannten Modulation ausgewertet wird.

## Claims

1. A spectrometer (10) for gas analysis, the spectrometer (10) comprising a measurement cell (28) having a gas (30) to be investigated; a light source (12) for the transmission of light (14) into the measurement cell (28) on a light path (16); a filter arrangement (22) having a Fabry-Perot filter (24a-c) in the light path (16), in order to set frequency properties of the light (14) by means of a transmission spectrum of the filter arrangement (22); a detector (36, 38) which measures the absorption of the light (14) by the gas (30) in the measurement cell (28), wherein the filter arrangement (22) comprises a plurality of Fabry-Perot filters (24a-c) and a control unit (44) for the filter arrangement (22) in order to change the transmission spectrum by setting at least one of the Fabry-Perot filters (24a-c),
**characterized in that**
the control unit (44) is configured to change the transmission spectra by setting the Fabry-Perot filters (24a-c) in accordance with a scheme of scanning once a transmission spectrum having a maximum at a scanning frequency is generated the amplitude of the maximum is modulated at the scanning frequency over a plurality of cycles and the scanning frequency is then systematically changed so that the spectrum is scanned successively at different frequencies with a series of pulses wherein the signal of the detector is evaluated with a Lock-In-method by means of the known modulation.

2. The spectrometer in accordance with claim 1,
wherein the filter arrangement (22) has three Fabry-Perot filters (24a-c).

3. The spectrometer in accordance with claim 1 or 2,
wherein an association of mirror spacings of the Fabry-Perot filters (24a-c) and of associated transmission spectra are stored in the control unit (44).

4. The spectrometer in accordance with claim 2 or3,
wherein the Fabry-Perot filters (24a-c) have graduated full widths at half maximum so that a Fabry-Perot filter with a low full width half maximum, a Fabry-Perot filter with a mean full width half maximum and a Fabry-Perot filter with a high full width half maximum is provided.

5. The spectrometer in accordance with any preceding claim,
wherein the amplitude is modulated with a delta pulse, a sinusoidal pulse or an exponentially growing and then abruptly falling-off pulse.

6. The spectrometer in accordance with any preceding claim,
further comprising an additional optical band pass filter (20) in the light path (16), in order to mask out maxima of higher orders of at least one of the Fabry-Perot filters (24a-c).

7. The spectrometer in accordance with any preceding claim,
wherein at least one of the Fabry-Perot filters (24a-c) is mounted such that it can be removed from the light path (16) and can be reintroduced into the light path (16).

8. A method of spectrometric gas analysis, in which light (14) of a light source (12) passes through a filter arrangement (22) having a Fabry-Perot filter (24a-c) and the frequency properties of the light (14) are set in this connection by the transmission spectrum of the filter arrangement (22), the light (14) subsequently being guided in a measurement cell (28) having a gas (30) to be investigated and the absorption of light (14) by the gas (30) in the measurement cell (28) is measured by means of a detector (36, 38) wherein the absorption is measured after each setting of one of a plurality of Fabry-Perot filters (24a-c) of the filter arragement (22) and in that way the absorption is measured with light of different frequency properties,
**characterized in that**,
the transmission spectra is changed by means of a control unit (44) by setting the Fabry-Perot filters (24a-c) in accordance with a scheme of scanning once a transmission spectrum having a narrow range maximum at a scanning frequency is generated, the amplitude of the maximum is modulated at the scanning frequency over a plurality of cycles and the scanning frequency is then systematically changed so that the spectrum is scanned successively at different frequencies with a series of pulses wherein the signal of the detector is evaluated with a Lock-In-method by means of the known modulation.

## Revendications

1. Spectromètre (10) pour l'analyse des gaz, le spectromètre (10) comprenant une cellule de mesure (28) ayant un gaz (30) à étudier; une source de lumière (12) pour la transmission de la lumière (14) dans la cellule de mesure (28) sur un trajet de lumière (16); un agencement de filtre (22) ayant un filtre de Fabry-Perot (24a-c) dans le chemin de lumière (16), afin de définir des propriétés de fréquence de la lumière (14) au moyen d'un spectre de l'agencement de filtre de transmission (22); un détecteur (36, 38) qui mesure l'absorption de la lumière (14) par le gaz (30) dans la cellule de mesure (28), dans lequel l'agencement de filtre (22) comprend une pluralité de Fabry-Perot filtre (24a-c) et une unité de commande (44) pour le dispositif de filtrage (22) afin de modifier le spectre de transmission en réglant au moins l'un des filtres Fabry-Pérot (24a-c),
**caractérisé en ce que**
l'unité de commande (44) est configurée pour modifier le spectre de transmission par le réglage des filtres Fabry-Pérot (24a-c) en conformité avec un schéma de balayage, selon lequel respectivement un spectre de transmission ayant un maximum à une fréquence de balayage est généré, l'amplitude du maximum est modulé à la fréquence de balayage sur une pluralité de cycles et la fréquence de balayage est alors systématiquement modifiés de sorte que le spectre est balayé successivement à différentes fréquences avec une série d'impulsions dans lequel le signal du détecteur est évaluée avec une méthode de blocage par moyen de la modulation connue.

2. Spectromètre selon la revendication 1,
dans lequel l'agencement de filtre (22) comporte trois filtres de Fabry-Perot (24a-c).

3. Spectromètre selon la revendication 1 ou 2,
dans lequel une association de miroirs espacements des filtres Fabry-Pérot (24a-c) et des spectres de transmission associée sont stockés dans l'unité de commande (44).

4. Spectromètre selon la revendication 2 ou 3,
dans lequel les filtres de Fabry-Perot (24a-c) ont des largeurs à mi-hauteur echelonnés de telle sorte qu'un filtre de Fabry-Perot avec une faible largeur maximale moitié plein, un filtre de Fabry-Perot avec une moyenne sur toute la largeur mi-hauteur et une cavité de Fabry-Perot filtrer avec une largeur moitié haute maximale est fourni.

5. Spectromètre selon l'une quelconque des revendications précédentes,
dans lequel l'amplitude est modulée avec une impulsion de delta, une impulsion sinusoïdale ou une impulsion se augmentant exponentiellement et ensuite tombant brusquement.

6. Spectromètre selon l'une quelconque des revendications précédentes,
comprenant en outre un filtre supplémentaire optique passe-bande (20) dans le trajet de lumière (16), afin de masquer des maxima d'ordres plus élevés d'au moins l'un des filtres Fabry-Pérot (24a-c).

7. Spectromètre selon l'une quelconque des revendications précédentes,
dans lequel au moins l'un des filtres Fabry-Pérot (24a-c) est monté de manière à pouvoir être retiré du trajet de la lumière (16) et peut être réintroduite dans le chemin de lumière (16).

8. Procédé d'analyse de gaz spectrométrique, dans lequel la lumière (14) d'une source de lumière (12) passe à travers un dispositif de filtrage (22) ayant un filtre de Fabry-Perot (24a-c) et les propriétés de fréquence de la lumière (14) sont fixées à cet égard par le spectre de l'agencement de filtre (22), la lumière (14) étant ensuite guidée dans une cellule de mesure (28) ayant un gaz (30) à étudier et l'absorption de la lumière (14) par le gaz (30) dans la cellule de mesure (28) est mesurée au moyen d'un détecteur (36, 38) dans lequel l'absorption est mesurée après chaque réglage de l'un d'une pluralité de filtres Fabry-Perot (24a-c) de l'arrangement de filtre (22) et en ce que le moyen d'absorption est mesuré avec une lumière de différentes propriétés de fréquence,
**caractérisé en ce que**,
le spectre de transmission est modifié au moyen d'une unité de commande (44) par le réglage des filtres Fabry-Pérot (24a-c) en conformité avec un schéma de balayage, selon lequel respectivement un spectre de transmission ayant un maximum à une fréquence de balayage est généré, l'amplitude du maximum est modulé à la fréquence de balayage sur une pluralité de cycles et la fréquence de balayage est alors systématiquement modifiés de sorte que le spectre est balayé successivement à différentes fréquences avec une série d'impulsions dans lequel le signal du détecteur est évaluée avec une méthode de blocage par moyen de la modulation connue.
